# EUROPÄISCHE PATENTSCHRIFT

(11) **EP 1 540 016 B1**
(45) Veröffentlichungstag und Bekanntmachung des Hinweises auf die Patenterteilung: **17.12.2008**
(21) Anmeldenummer: 03792143.4
(22) Anmeldetag: 15.08.2003
(51) Int. Cl.: C12Q 1/68

(54) **VERFAHREN ZUR BESTIMMUNG DES ALLELISCHEN ZUSTANDES AM 5'-ENDE DES ALPHA-S1-KASEINGENS**
METHOD FOR DETERMINING THE ALLELIC STATE OF THE 5'-END OF THE alpha-S1-CASEIN GENE
PROCEDE PERMETTANT DE DETERMINER L'ETAT ALLELIQUE DE LA TERMINAISON 5' DU GENE DE LA CASEINE alpha-S1

(30) Priorität: 16.08.2002 DE 10238433
(43) Veröffentlichungstag der Anmeldung: 15.06.2005
(73) Patentinhaber: Justus-Liebig-Universität Giessen, 35390 Giessen (DE)
(72) Erfinder: PRINZENBERG, Eva-Maria, 22927 Grosshansdorf (DE); ERHARDT, Georg, 35415 Pohlheim (DE)
(74) Vertreter: Meyer-Dulheuer, Karl-Hermann
(86) Internationale Anmeldenummer: PCT/DE2003/002747
(87) Internationale Veröffentlichungsnummer: WO 2004/018696

(56) Entgegenhaltungen:
- US-A- 4 873 316
- KOCZAN D ET AL: "GENOMIC ORGANIZATION OF THE BOVINE ALPHA-S1 CASEIN GENE" NUCLEIC ACIDS RESEARCH, OXFORD UNIVERSITY PRESS, SURREY, GB, Bd. 19, Nr. 20, 1991, Seiten 5591-5596, XP002915050 ISSN: 0305-1048
- ANIMAL SCIENCE PAPERS AND REPORTS, Bd. 17, Nr. 1, 1999, Seiten 29-33, XP0001179556 ISSN: 0860-4037
- JOURNAL OF ANIMAL AND FEED SCIENCES, Bd. 9, Nr. 1, Januar 2000 (2000-01), Seiten 73-79, XP0001179557 ISSN: 1230-1388
- THEORETICAL AND APPLIED GENETICS, Bd. 93, Nr. 5-6, 1996, Seiten 887-893, XP0009026049 ISSN: 0040-5752
- JOURNAL OF ANIMAL BREEDING AND GENETICS, Bd. 114, Nr. 2, 1997, Seiten 121-132, XP0009026048 ISSN: 0931-2668
- DATABASE EMBL [Online] XP002273453 gefunden im HTTP://WWW.EBI.AC.UK/ Database accession no. X59856
- GENE (AMSTERDAM), Bd. 126, Nr. 2, 1993, Seiten 213-218, XP0001179551 ISSN: 0378-1119
- JOURNAL OF DAIRY SCIENCE, Bd. 70, Nr. 12, 1987, Seiten 2585-2598, XP0009026144 ISSN: 0022-0302
- ANIMAL GENETICS. FEB 1993, Bd. 24, Nr. 1, Februar 1993 (1993-02), Seite 74, XP0009026055 ISSN: 0268-9146
- ARCHIV FUER TIERZUCHT, Bd. 39, Nr. 4, 1996, Seiten 369-385, XP0009026150 ISSN: 0003-9438
- PRINZENBERG E.-M.: "Kapitel 2.4: "Züchterische Bedeutung genetisch bedingter Milchproteine"" 1998, FACHVERLAG KÖHLER , XP0001179534 ISBN-3-922306-68-3 Seiten 14-21
- PRINZENBERG E.-M.: "Kapitel 4.1: "alphaS1-Kaseingen"" 1998, FACHVERLAG KÖHLER , XP0001179535 ISBN 3-922306-68-3 Seiten 61-73

## Beschreibung

Die Erfindung betrifft einen genetischen Marker am 5'-Ende des αS1-Kaseingens (*CSN1S1*) und des Kaseingen-Komplexes und ein Verfahren zur alters- und laktationsunabhängigen Typisierung von Rindern durch Bestimmung des allelischen Zustands in diesem Bereich, sowie die Verwendung dieses Verfahrens zur Auswahl von Organismen mit einem bevorzugten Allel, beispielsweise in der markergestützten Selektion.

### Stand der Technik

Das Vererbungspotential (im Hinblick auf den Milchproteingehalt und andere züchterisch relevante Merkmale) von Zuchttieren wird derzeit mittels der Zuchtwertschätzung anhand von Testpaarungen und Leistungserfassung der Nachkommen abgeschätzt. Der Nachteil dieses konventionellen Verfahrens ist offensichtlich, beim Rind vergehen zwischen der ersten Besamung mit einem Testbullen und dem Einsetzen der Laktation der ersten Töchter ca. 3 Jahre, bis zur Erfassung einer kompletten Laktation der Tochter also ca. 4 Jahre. Erst danach kann der Zuchtwert abgeschätzt werden. Bis dahin entstehen durch die Haltung der Bullen bis zum Vorliegen der ersten geschätzter Zuchtwerte und den Testpaarungen Kosten, die über diesen langen Zeitraum und in der Summe der Tiere erheblich sind. Für die Erfassung der Eigenleistung und Ermittlung eines Zuchtwertes bei Kühen gilt dies analog.

Daher werden seit einigen Jahren international Anstrengungen unternommen, mit Hilfe der Fortschritte in der Genomanalyse genetische Marker und direkte Gentests für züchterisch relevante Leistungsparameter zu entwickeln. Genomweite Markeranalysen haben dabei mit Hilfe der Kopplungsanalyse die Eingrenzung von Chromsomenbereichen, in denen leistungsbestimmende Genorte liegen - sogenannte QTL-Regionen (QTL = Quantitative Trait Loci) - ermöglicht. Derartige QTL-Studien und daraus resultierende Tests sind unter anderem in der WO 20000 36143 und der WO 2001 57250 A2/A3 beschrieben. Weitere Details der QTL-Analyse beim Nutztier und ein Verfahren, mit dem basierend auf QTL-Studien auch ursächliche Kandidatengene isoliert werden können beschreibt die DE 100 17 675 A1, deren Offenbarungsgehalt hier mit einbezogen wird.

Beim Rind und anderen zur Milchproduktion gezüchteten Spezies stellen die Milchmenge, Proteingehalte und Fettgehalte die entscheidenden Kriterien dar. Für diese Merkmale sind verschiedene QTL, unter anderem auf dem Chromosom BTA 6 identifiziert worden. Die potentiellen QTL-Regionen für Proteingehalte werden von verschiedenen Arbeitsgruppen relativ einheitlich mit dem Bereich um oder zwischen den Mikrosatellitenmarkern *BM143* und *TGLA37* und damit rund 20-30 Centimorgan (cM) vom Kaseinlocus entfernt angegeben (Spelman et al. 1996, Genetics 144, 1799-1808; Georges et al. 1995, Genetics 139, 907-920; Kühn et al. 1996, J Anim Breed Genet 133, 355-362; Zhang et al. 1998, Genetics 149, 1959-1973). Nach Nadesalingam et al. (2001, Mammalian Genome 12, 27-31) sind allerdings die Kaseingene aufgrund ihren Position (40cM entfernt vom QTL) als Kandidat für die beobachteten QTL-Effekte ebenfalls ausgeschlossen.

Bereits seit Mitte der 80er Jahre wurden auch die genetisch bedingten Milch proteinvarianten des Rindes im Hinblick auf einen Einfluss auf Milchmengen- und

Milchqualitätsmerkmale untersucht. Dies erfolgte teils mittels Erfassung der phänotypisch (in der Milch) unterscheidbaren Proteinvarianten (Ng-Kwai-Hang et al., 1984, J Dairy Sci 67, 835-840 und Ng-Kwai-Hang et al., 1986, J Dairy Sci 69, 22-26,), später auch mittels molekulargenetischer Verfahren, die die den Proteinvarianten zugrundeliegenden genetischen Mutationen nachwiesen (Sabour et al., 1996, J Dairy Sci 79, 1050-1056). Die bisherigen Untersuchungen zeigen dabei teilweise widersprüchliche Effekte der untersuchten Varianten, die sich zwischen Rassen und regionalen Herkünften nicht immer bestätigen lassen (zusammengefasst bei Prinzenberg, 1998, ISBN 3-922306-68-3, Kap 2.4, S. 14-21). Die Mehrzahl dieser Untersuchungen konzentriert sich auf die Varianten des β-Laktoglobulins, des β- und κ-Kaseins, da im αs1-Kasein nur zwei Proteinvarianten in nennenswerter Häufigkeit vorkommen und insbesondere in den bereits stark selektierten Milchrassen wie Holstein Friesian / Deutsche Holstein nahezu ausschliesslich die Proteinvariante αs1-Kasein B zu finden ist (Ng-Kwai-Hang et al., 1990, J Dairy Sci 73, 3414-3420; Erhardt et al., 1993, J Animal Breed Genet 36, 145-152; Lien et al., 1999, Animal Genetics 30, 85-91). In einer neueren Untersuchung an Milchrindern mit unterschiedlichem Holstein Blutanteil (Freyer et al., 1999, J Animal Breed Genet 116, 87-97) wurde αs1-Kasein in den Kopplungsanalysen ebenfalls nicht verwendet, da keine ausreichende Variabilität vorhanden war.

Zur molekulargenetischen Differenzierung der αs1-Kaseinvarianten B und C sind verschiedene Tests beschrieben (David & Deutch 1992, Animal Genetics 23, 425-429; Schlee & Rottmann 1992, J Anim Breed Genet 109, 316-319). Für die seltenen Allele A, D und F existieren ebenfalls einzelne Gentestverfahren (Prinzenberg 1998, ISBN 3-922306-68-3; Kap Kap 4.1, S 61-71), wie auch für den Nachweis einer quantitativen Variante αs1-Kasein G (Mariani et al 1995, L'industria del Latte 31, 3-13). Schild & Geldermann (1996) zeigten mittels Sequenzierung von rund 1000 Basenpaaren (bp) aus dem 5'-Bereich des αs1-Kaseingens bei verschiedenen Rinderrassen 17 variable Positionen im 5'-flankierenden Bereich des *CSN1S1* Gens, von denen 5 aufgrund variabler Erkennungssequenzen für Restriktionsendonukleasen mit Restriktionsfragmentlängenpolymorphismen (PCR-RFLP) nachweisbar waren. Nach Ehrmann et al. (1997, J Animal Breed Genet 114, 121-132) sind die 5'-flankierenden Varianten jeweils mit bestimmten Proteinallelen gekoppelt, so dass von den vorhandenen Proteinvarianten auch auf bestimmte Varianten im 5'-flankierenden Bereich geschlossen werden kann. Einen Gentest zur Unterscheidung von αs1-Kasein B und C bei Deutschen Schwarzbunten, Fleckvieh und Jersey-Kühen, welcher auf einem Fragment aus dem 5'-flankierenden Bereich des αs1-Kaseingens basiert, beschrieben auch Koczan et al. (1993, Animal Genetics 24, 74). Für den letztgenannten Test wurde die strikte Kopplung mit den Proteinvarianten αs1-Kasein B und C und damit die Gültigkeit für die Rassen Aberdeen Angus, Anatolisches Schwarzvieh, Angler, Asturian Valley, Ayrshire, British Frisian, Casta Navarra, Charolais, Chianina, Fighting Bull, Hereford, Jersey, Maremmana, Pezzata Rossa, Piemonteser, Scottish Highland, Türkisches Graues Steppenrind inzwischen jedoch widerlegt (Jann et al., 2001; Arch. Tierz, Dummerstorf 45, 13-21).

Die Kaseingene sind als eng gekoppelter Genlocus beim Rind und beim Schaf auf Chromosom 6, beim Menschen auf Chromosom 4 und bei der Maus auf Chromosom 5 kartiert. Auch für andere Tierarten (Kaninchen, Schwein, Ziege) ist die Kopplung der Kaseingene nachgewiesen. Aufgrund dieser engen Kopplung, ist die derzeitige Angabe der Lokalisation des αs1-Kaseingens in genetischen Karten beim Rind an die Lokalisation des κ-Kaseingens gebunden. Für beide Gene wird in der aktuellen Genkarte des Rindes die physische Position BTA6q31-33 und die genetische Position 82,6 cM (MARC97) bzw. 103,0 cM (IBRP97) angegeben. Die Rekombinationsrate zwischen αs1-Kasein- und κ-Kaseingen wird damit als Null angenommen.

Die Nutzung einer Lactalbuminsequenz für die Auswahl von Zuchttieren ist in der EP0555435 offenbart. Für das bovine κ-Kasein existieren ebenfalls zahlreiche Gentests (Denicourt et al., 1990, Animal Genetics 21, 215-216; Medrano & Aguilar-Cordova, 1990, Biotechnology 8, 144-145; Pinder et al., 1991, Animal Genetics 22, 11-20; Schlee & Rottmann, 1992, J Animal Breed. Genet. 109, 153-155; Zadworny & Kuhnlein, 1990, Theor. Appl. Genet. 80, 631-634), da diesem Protein Einflüsse auf die Verarbeitungseigenschaften und die Käsereieignung der

Milch zugeschrieben werden (siehe Lodes et al., 1996, Milchwissenschaft 51, 368-373 und 543-548).

Aufgrund der milchdrüsenspezifischen Expression werden die Promotoren der bovinen Milchproteingene und die des αs1-Kaseingens auch bei der Erstellung von transgenen Tieren und zur Expression in Zellkulturen genutzt. Die DE 38 54 555 T2, deren Offenbarungsgehalt hier mit einbezogen wird, beschreibt die Verwendung des αs1-Kaseinpromotors und des Signalpeptids zur Produktion von rekombinanten Proteinen in der Milch von Säugetieren. Einen Überblick über die Nutzung von transgenen Tieren zur Produktion von rekombinanten Proteinen und die dazu verwendeten Promotoren gibt auch Rudolph (1999, Trends in Biotechnology (TIBTECH) 17, 367-374).

Einen direkten Gentest für ein Gen aus dem Fettsäurestoffwechsel (*DGAT1*) beschreiben Winter et al. (2002, PNAS 99, 9300-9305) und schreiben diesem Gen einen Effekt auf den Milchfettgehalt zu.

Der Nachteil aller Verfahren, die basierend auf einer Milchprobe die genetischen Varianten phänotypisch (d.h. in Milchproben) differenzieren besteht darin, dass nur laktierende Kühe untersucht werden können. Daher besteht der Bedarf, die Tiere laktationsunabhängig untersuchen zu können. Weiterhin stellen die im codierenden Bereich der Milchproteingene nachgewiesenen Polymorphismen nach derzeitigem Stand der Technik keine zuverlässigen Marker für Milchleistungsmerkmale dar. Die bisherigen QTL-Analysen weisen auf einen ausserhalb der Milchproteingene liegenden QTL hin.

Für αs1-Kasein ist derzeit kein Marker mit ausreichender Variabilität vorhanden, so dass sich dieses Gen einer näheren Analyse von Effekten auf Milchleistungs- und Inhaltsstoffmerkmale weitgehend entzieht. Alle vorhandenen Testverfahren beruhen auf der molekulargenetischen Differenzierung der auch phänotypisch vorhandenen Variation.

Die Mikrosatellitenmarker, welche bei QTL-Analysen ermittelt wurden, eignen sich nur bedingt zum Einsatz in der markergestützten Selektion, da die jeweilige Marker-QTL-Kopplung zunächst geklärt werden muss. Es handelt sich bei diesen Mikrosatellitenmarkern jeweils um indirekte Tests, die je nach Dichte der Kopplung zum ursächlichen Genort eine reduzierte Aussagesicherheit haben.

Der Nachteil des Verfahrens der EP 0555435 besteht darin, dass α-Lactalbumin nur einen geringen (ca. 2-5%) Anteil des gesamten Milcheiweiss ausmacht. Den größten Anteil stellen die Kaseine (αs1-, αs2-, β- und κ-Kasein) mit rund 80% am Gesamteiweiss dar. Daher ist bei Anwendung dieses Selektionsmarkers nur ein geringer züchterischer Fortschritt zu erwarten.

Der Gentest für *DGAT1* von Winter et al. hat den Nachteil, dass aus Sicht der Züchter und der Milcherzeuger der Fettgehalt der Milch nicht das primäre Interesse geniesst, sondern hinter dem Proteingehalt zweitrangig ist.

Der Nachteil des Verfahrens der DE 38 54 555 T2 besteht darin, dass der verwendete Anteil des αs1-Kaseinpromotors nicht näher anhand einer Nukleotidsequenz charakterisiert ist. Es wird ein 9kb Fragment mit den Exons I und II, welches durch die Schnittstellen für *Kpn*I und *Bam*HI flankiert wird, verwendet. Es erfolgt keine Berücksichtigung der genauen Basenfolge oder von möglichen Variationen, die die Effektivität der Expression mit diesem Abschnitt des Promotors beeinflussen können.

Der Nachteil des Testverfahrens von Koczan et al. (1993, Animal Genetics 24, 74) besteht darin, dass zum einen die Zuverlässigkeit der Differenzierung zwischen den Varianten αs1-Kasein B und C (für die der Test entwickelt wurde) inzwischen widerlegt wurde, zum anderen ist mit dem Verfahren nur eine einzige Position der Basensubstitution nachweisbar und weitere Mutationen in diesem Bereich bleiben unberücksichtigt.

Derzeit gibt es keinen zuverlässigen Marker für Milchproteingehalt und keinen direkten genetischen Test für einen funktionalen Genabschnitt, um Tiere alters- und laktationsunabhängig auf ihr genetisches Potential hin zu testen.

### Aufgabe der Erfindung

Aufgabe der Erfindung ist es daher einen genetischen Marker und ein Verfahren zur Typisierung auf Milchleistungsmerkmale bereitzustellen, um Tiere alters- und laktationsunabhängig auf Milchleistungsmerkmale anhand ihres genetischen Materials zu untersuchen.

Die Lösung der Aufgabe erfolgt durch Bereitstellung eines auch in selektierten Milchrassen polymorphen genetischen Markers in der αs1-Kaseingenregion und eines Verfahrens, das eine alters- und laktationsunabhängige Typisierung der Tiere, die genetische Kartierung des αs1-Kaseingens, die Untersuchung von eng an diesen Genort gekoppelten oder direkt dadurch hervorgerufenen Effekten und eine züchterische Nutzung ermöglicht.

Bei dem erfindungsgemäßen Verfahren gemäß Anspruch 7 handelt es sich um einen genetischen Test für einen funktionalen Genabschnitt, die Aussagesicherheit ist größer als bei gekoppelten Markern und das Test-Ergebnis liegt innerhalb weniger Tage bis hin zu Stunden vor. Anhand dieses Tests können auch Zuchttiere ausgewählt werden, die mit höherer Wahrscheinlichkeit positive Vererbungseigenschaften haben, so dass die Anzahl Testpaarungen und zu testenden Tiere verringert werden können, wodurch die erheblichen Kosten der Testpaarungen reduziert werden können. Das erfindungsgemäße Verfahren beseitigt somit die beschriebenen Nachteile im Stand der Technik.

Mit dem erfindungsgemäßen Marker gemäß Anspruch 1 ist auch die Auswahl von besonders vorteilhaften Promotoren zur Erzeugung von Expressionsvektoren und transgenen Tieren möglich.

In einer besonders vorteilhaften Ausführungsform besteht die Erfindung aus einem Testkit, der Primer gemäß Seq ID No:1 und Seq ID No:2 zur Anreicherung eines Teilbereiches der Markersequenz des αs1-Kaseingens sowie Referenzproben für eine oder mehrere Sequenzen der Markersequenz des αs1-Kaseingens und dessen Allele enthält.

Folgende Abbildungen sind der Beschreibung beigefügt:
**Abbildung 1** DNA-Sequenz aus dem 5'-flankierenden Bereich des αs1-Kaseingens, im folgenden als Markersequenz bezeichnet.
**Abbildung 2** Alignment der Nukleinsäuresequenzen der allelischen Zustände des αs1-Kaseingens Allel 1, Allel 2, Allel 3, Allel 4 (Unterschiede in potentiellen Transkriptionsfaktor-Bindungsstellen sind hervorgehoben)
**Abbildung 3** Schematische Darstellung der Wanderungsmuster der Allele 1 bis 4 des Markers *CSN1S1* in der SSCP-Analyse
**Abbildung 4** Ergebnis der Varianzanalyse

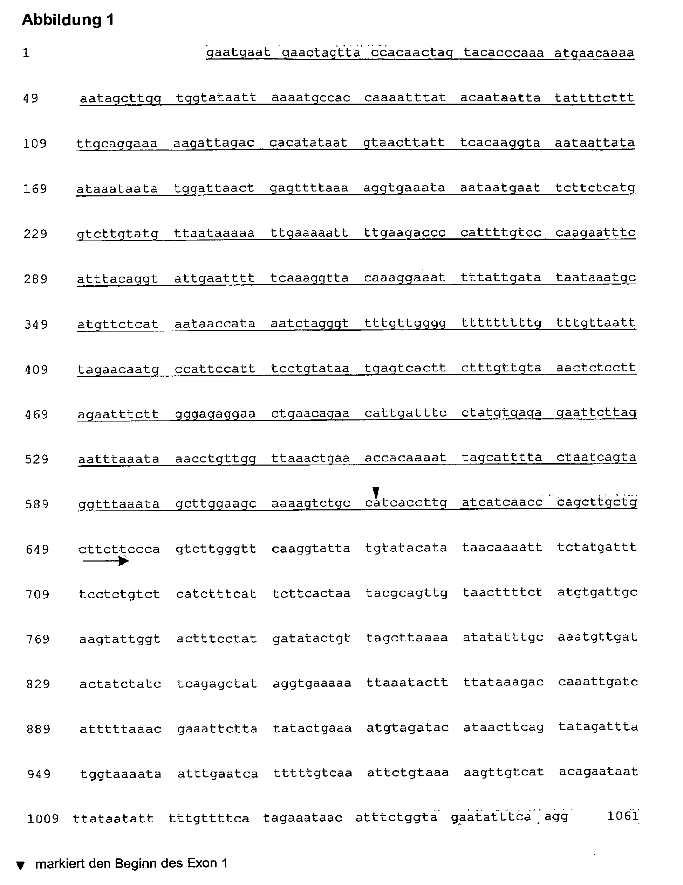

Überraschender weise wurde gefunden, dass der untersuchte Sequenzabschnitt, welcher durch die Oligonukleotide CSN1S1pro1f und CSN1S1pro1r bzw. CSN1S1pro2r begrenzt wird (grauer Kasten in Abbildung 1) innerhalb der Rasse Deutsch Holstein vier mittels einer Einzelstrang-Konformationspolymorphismen-Analyse detektierbare Allele aufweist und damit ausreichend polymorph ist, um eine genetische Kartierung und Analysen zum Effekt der Allele auf Milchleistungsparameter durchzuführen.

Es handelt sich dabei um einen 1061 bp großen Abschnitt aus dem 5'-flankierenden Bereich und des Exon 1 (siehe Abbildung 1), im besonderen um den 654 bp großen Abschnitt, der durch die beiden Oligonukleotide CSN1S1pro1f und CSN1S1pro1r begrenzt wird.

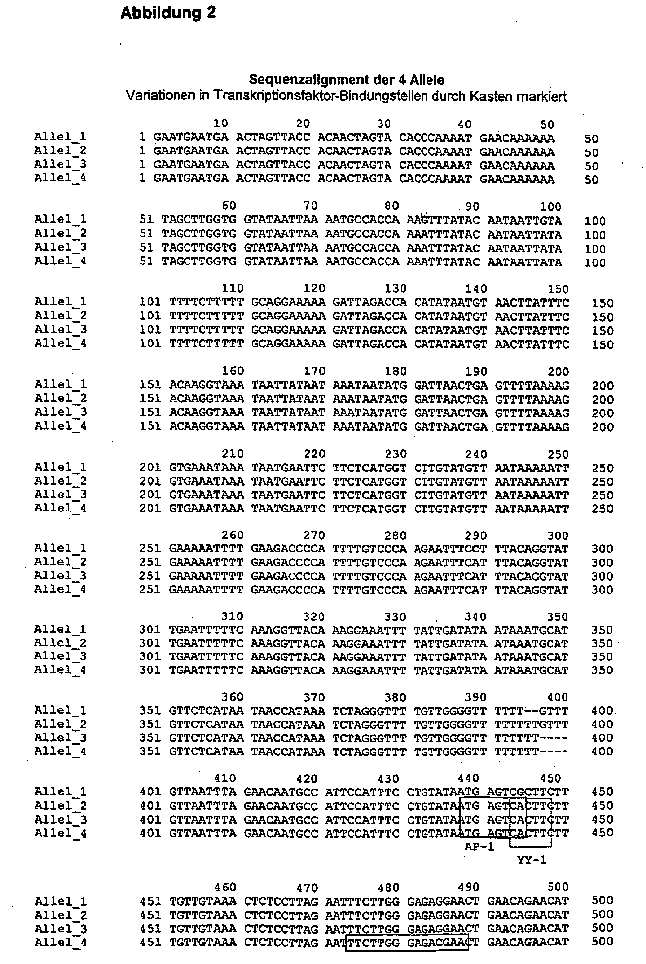

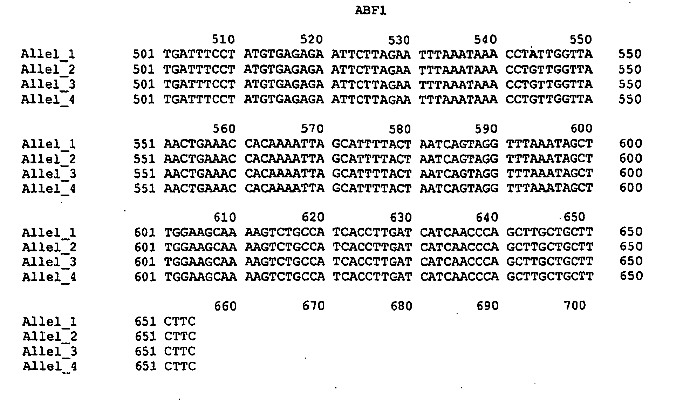

Die vier Allele wurden kloniert und sequenziert. Die Sequenzanalyse zeigt bis auf die Länge des poly-T (ab Position 390 der Abbildung 1) Übereinstimmung von Allel 2 mit der von Koczan et al. (1991, Nucleic Acids Research 19, 5591-56596; Genbank Acc. No. X59856) publizierten Sequenz. Die Allele 1, 3 und 4 unterscheiden sich durch verschiedene Substitutionen und Deletionen von dieser Sequenz. Die variablen Positionen sind im Sequenzalignment (Abbildung 2) hervorgehoben. In den Allelen 1 und 4 sind jeweils potentielle Transkriptionsfaktor-Bindungsstellen durch Mutationen betroffen. Im Allel 1 fallen demnach zwei potentielle Bindungsstellen (für AP-1 und YY1) weg, wohingegen im Allel 4 eine potentielle ABF1-Bindungsstelle neu entsteht.

Der gefundene Polymorphismus ist damit in einer vermutlich funktionalen Genregion lokalisiert und somit ein geeigneter Marker für Milchleistungsmerkmale, insbesondere für den Proteingehalt.

Der Sequenzabschnitt wird mit folgenden Oligonukleotidsequenzen flankiert, die als Primer für die Amplifikation mittels PCR verwendet werden, wobei die Kombinationen Primer 1 mit Primer 2 und Primer 1 mit Primer 3 möglich sind:
Primer 1: CSN1S1pro1f (5' GAA TGA ATG AAC TAG TTA CC 3')
Primer 2: CSN1S1pro1r (5' GAA GAA GCA GCA AGC TGG 3')
Primer 3: CSN1S1pro2r (5' CCT TGA AAT ATT CTA CCA G 3')

Die Primerbindungsstellen sind in der Abbildung 1 grau unterlegt.

Es wird ein erfindungsgemäßes Verfahren bereitgestellt, das direkt am Erbmaterial des zu untersuchenden Organismus durchgeführt werden kann. Mit Hilfe des erfindungsgemäßen Markers wird eine genetische Kartierung des αs1-Kaseingens innerhalb der Kopplungskarte ermöglicht und die Ermittlung des allelischen Zustands bei einzelnen Organismen, z.B. Rindern, vorgenommen, die innerhalb weniger Stunden das genetische Potential im Hinblick auf Milchproteingehalt ermittelt.

Das Verfahren zur Ermittlung des genetischen Potentials im Hinblick auf Milchproteingehalt durch Ermittlung des allelischen Zustands des erfindungsgemäßen Markers besteht im Einzelnen aus:
1. Bereitstellung des genetischen Materials des zu untersuchenden Organismus, eines männlichen oder weiblichen Zuchtrindes oder eines Embryos.
   Der Organismus ist dabei definitionsgemäß ein Tier, insbesondere ein Säugetier, im besonderen ein Rind, ein Schaf oder eine Ziege einschließlich Embryonen dieser Spezies.
   Der Organismus ist auch ein gentechnisch veränderter Organismus (GVO), welcher den beschriebenen Sequenzabschnitt aus dem αs1-Kaseingen und des 5'-flankierenden Bereich (Abbildung 1) oder Teile dessen enthält.
   Das genetische Material ist definitionsgemäß genomische DNS oder RNS von Tieren, aber auch Plasmid-DNS aus Bakterien, von artifiziellen Chromosomen wie BACs und YACs oder für spezielle Anwendungen erstellte Konstrukte aus genetischem Material verschiedener Organismen, z.B. zur Herstellung von Transgenen.
   Das Ausgangsmaterial zur Gewinnung von DNS- oder RNS-haltigem Material ist z.B. Blut, Leukozyten, Gewebe einschließlich Biopsiematerial, Milch, Sperma, Haare, einzelne Zellen einschließlich Zellmaterial aus Embryonen, eine Bakterienkultur oder auch isolierte Chromosomen. Weiterhin ist auch bereits zuvor amplifiziertes genetisches Material, welches die Markersequenz (Abbildung 1) oder Teile daraus enthält, erneut Ausgangsmaterial.
2. Gezielte Isolierung oder Anreicherung des Sequenzabschnitts der Abbildung 1 oder einer Sequenz, die Teilbereiche davon enthält, vorzugsweise den dargestellten Sequenzabschnitt Position 1 bis 655 der Abbildung 1.
   Die Isolierung des genetischen Materials erfolgt nach Standardmethoden, wie sie z.B. im Handbuch "Molecular Cloning" (Sambrook, Fritsch, Maniatis, 1989; Cold Spring Harbour Laboratory Press, New York) beschrieben sind oder kann mittels kommerziell erhältlicher Kits (z.B. Nucleospin, Machery Nagel, Düren, Deutschland) durchgeführt werden.
   Die Anreicherung erfolgt vorzugsweise mittels Polymerase Kettenreaktion (PCR, Mullis & Falloona, 1987, Methods in Enzymology 155, 335-350), wobei auch fluoreszenzmarkierte, radioaktiv oder chemisch markierte Primer eingesetzt werden können. Bei Verwendung von RNS als genetisches Material wird zweckmäßigerweise eine vorherige reverse Transkription (Myers & Gelfand 1991, Biochemistry 30, 7661-7666*)* durchgeführt.
   Der Sequenzabschnitt wird vorzugsweise mit folgenden Oligonukleotidsequenzen als Primer für die Amplifikation angereichert, wobei die Kombinationen Primer 1 mit Primer 2 und Primer 1 mit Primer 3 möglich sind:
   Primer 1: CSN1S1pro1f (5' GAA TGA ATG AAC TAG TTA CC 3')
   Primer 2: CSN1S1pro1r (5' GAA GAA GCA GCA AGC TGG 3')
   Primer 3: CSN1S1pro2r (5' CCT TGA AAT ATT CTA CCA G 3')

   Die Auswahl weiterer Primer, die die Amplifikation einer Teilsequenz der in Abbildung 1 beschriebenen Sequenz ermöglicht, innerhalb derer variable Nukeotidpositionen zur Unterscheidung der Allele 1 bis 4 liegen, ist ausdrücklich möglich.
3. Nachweis des allelischen Zustands im isolierten oder angereicherten Sequenzbereich der Abbildung 1, vorzugsweise innerhalb der Teilsequenz, die durch CSN1S1pro1f und CSN1S1pro1r begrenzt wird.
   Zur Bestimmung des allelischen Zustands stehen eine Reihe von Standard-Techniken, die dem Fachmann bekannt sind, zur Verfügung, wie die Sequenzierung nach Sanger et al. 1977, das Verfahren des Pyrosequencing (www.pyrosequencing.com), durch Darstellung von Einzelstrang Konformationspolymorphismen (SSCP, Orita et al. 1989, Genomics 5, 874-879), mittels Restriktionsfragment Längenpolymorphismen (RFLP; Botstein et al. 1980, American Journal of Human Genetics 32, 314-331) und PCR-RFLP (Damiani et al. 1990, Animal Genetics 21, 107-114; Medrano & Aguilar-Cordova 1990, Animal Biotechnology 1,73-77), allelspezifischer PCR (= ARMS, ASPCR, PASA; Newton et al. 1989, Nucleic Acids Research 17, 2503-2516; Sakar et al. 1990, Analytical Biochemistry 186, 64-68; David & Deutch 1992, Animal Genetics 23, 425-429), Oligonukleotid-Ligations-Test (= OLA; Beck et al. 2002, J Clinical Mikrobiol 40, 1413-1419), Temperaturgradienten Gel Elektrophorese (= TGGE, Tee et al. 1992, Animal Genetics 23, 431-435) und analoge, zum Stand der Technik gehörende Verfahren.
   Es wird vorgeschlagen, die Primer mit einer Markierung (Fluoreszenz, Radioaktivität und ähnliches) zu versehen und die Bestimmung des allelischen Zustands am Sequenzierautomaten, durch Autoradiografie oder Chemilumineszenz durchzuführen. Bei Verwendung nicht-markierter Primer erfolgt die Bestimmung des allelischen Zustands durch Darstellung der Fragmente nach Gelelektrophorese durch Färbung der Nukleinsäuren, z.B. mit Ethidiumbromid (Sambrook et al., 1989) oder im Silberfärbeverfahren (Bassam et al 1991, Analytical Biochemistry 196, 80-83).
   Weiterhin ist es möglich, verschiedene Hochdurchsatzverfahren zum Mutationsnachweis, darunter die Verwendung von Oligonukleotid-Arrays (Dong et al 2001, Genome Research 11, 1418-1424), das TaqMan-Verfahren (Ranade et al 2001, Genome Research 11, 1262-1268), Fluoreszenz-Polarisationsverfahren (Chen et al 1999, Genome Research 9, 492-498), Massenspektrometrische Verfahren (MALI-TOF; Sauer et al. 2002, Nucleic Acids Research 30, e22) einzusetzen. Diese Aufzählung ist beispielhaft und nicht limitierend zu verstehen.
   Der allelische Zustand ist dabei als das Vorhandensein einer bestimmten Nukleotidsequenz innerhalb des angereicherten Bereiches zu verstehen. Abbildung 2 zeigt beispielhaft die Nukleinsäuresequenz von vier verschiedenen allelischen Zuständen des erfindungsgemäßen Markers (Abbildung 2, Allele 1, 2, 3 und 4).
   Im Falle der Sequenzierung muss ein Vergleich mit den korrespondierenden Nukleotidsequenzen in Abbildung 2 1, 2, 3 und 4 erfolgen, um die zu Typ Allel 1 bis Allel 4 analogen Zuordnungen vorzunehmen. Basierend auf den angegebenen Nukleotidsequenzen ist es einer mit dem Stand der Technik vertrauten Person auch möglich, die benötigten Restriktionsenzyme und Fragmentlängen bei einer PCR-RFLP Analyse zu bestimmen oder Oligonukleotide zum Nachweis über allelspezifische PCR (ASPCR) zu konzipieren. Auch die Anpassung der weiteren zuvor genannten Techniken zum Mutationsnachweis ist dem Fachmann möglich.
   Besonders vorteilhaft ist die Darstellung der allelischen Zustände mittels Einzelstrang-Konformationspolymorphismen (SSCP), da der allelische Zustand direkt anhand des Fragmentmusters abzulesen ist. Das Verfahren ermöglich zusätzlich zur Detektion der hier beschriebenen 4 Allele auch die Erkennung von weiteren, hier nicht beschriebenen Mutationen. Aus diesem Grund eignet es sich besonders gut auch zur Analyse des homologen Genombereiches bei anderen Tierarten als beim Rind. Um die Dauer der Gelelektrophorese zu reduzieren, ist die Verwendung eines kürzeren Fragmentes beispielsweise die in Abbildung 1 mit Pfeil markierte Sequenz, die durch die Oligonukleotide festgelegt wird, als der kompletten Sequenz empfehlenswert.

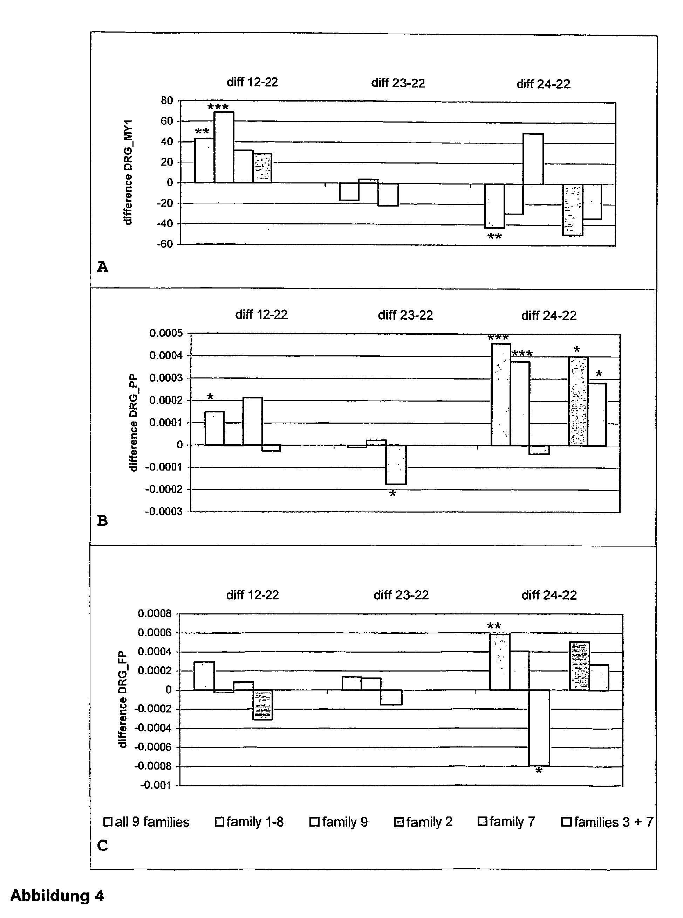

   Abbildung 4 zeigt eine schematische Darstellung der Allele 1 bis 4 des Markers CSN1S1 in der SSCP-Analyse im 12%igen Acrylamid:Bisacrlymid 49:1 Gel mit 1% Glycerolzusatz. Die Felder 1 bis 4 repräsentieren die vier verschiedenen Auftrennungsmuster der Allele. Die Wanderungsrichtung der Moleküle im elektrischen Feld von Kathode (-) zur Anode (+) ist mit einem Pfeil dargestellt. Die Einzelstränge der Allele zeigen ein typisches, deutlich voneinander verschiedenes Auftrennungsmuster. Da mittels Silberfärbung beide DNS-Einzelstränge dargestellt werden, ist jedes der Allele durch zwei Banden charakterisiert.
4. Auswahl von Organismen, die den jeweils vorteilhaften allelischen Zustand des erfindungsgemässen Markers tragen. Dies kann z.B. der allelische Zustand 1 oder 4 sein, welcher sich von Allel 2 durch die Anzahl der potentiellen Bindungsstellen für Transkriptionsfaktoren unterscheidet.

### Ausführungsbeispiele

### 1. Verfahren zur Typisierung auf Milchleistungsmerkmale durch Ermittlung des allelischen Zustands des erfindungsgemäßen Markers

Als Ausgangsmaterial wird Rinderblut verwendet. Die Isolierung des genetischen Materials (genomische DNS) erfolgt nach der Hochsalzmethode von Montgomery & Sise (1990, NZ J Agric Res 33, 437-441).

Für die Durchführung der Amplifizierung des Markers mittels PCR-Reaktion werden die folgenden Oligonukleotidsequenzen als Primer verwendet:
Primer 1 CSN1S1pro1f (5' GAA TGA ATG AAC TAG TTA CC 3')
Primer 2 CSN1S1pro1r (5' GAA GAA GCA GCA AGC TGG 3')
Die Reaktionsansätze enthalten in 15µl jeweils 20-100 ng zu testende genomische DNS, 10pmol jedes Oligonukleotids CSN1S1pro1f und CSN1S1pro1r, 0,5 U Taq-DNA-Polymerase (Peqlab Biotechnologie, Erlangen), 50 µM dNTPs in einem Standardpuffer (10mM Tris-HCl ph 8,8, 50 mM KCI, 1.5mM MgCl₂). Das Temperaturprogramm (in einem Thermocycler Modell iCycler der Firma Biorad) wird wie folgt gewählt: 1 min. - 93°C (1x), (40 sec - 91 °C, 40 sec. 57°C, 40 sec - 70°C) (30x) und 3 min - 70°C (1x). Danach erfolgte die Kühlung auf 4°C.

Jedem Reaktionsansatz werden anschließend je 25µl eines Formamid-Denaturierungspuffers zugegeben (95% Formamid, 0,025% (w/v) Bromphenolblau, 0,025% (w/v) Xylencyanol (FF), 20 mM EDTA), die Mischung für 2 min bei 93°C erhitzt, in Eiswasser abgekühlt und je 4µl der Mischung auf ein 12%iges 49:1 Acrylamid-Bisacrlymidgel mit 1 % Glycerolzusatz geladen. Die Auftrennung erfolgt über 20h bei 420V und 10°C in einer Vertikalelektrophoresekammer Modell Pengiun P9DS (OWL Scientific, Woburn, USA) mit einem 0,8 mm dünnen 16x16cm großen Gel. Als Lauf- und Gelpuffer wurde 0,5x TBE verwendet. Nach der Elektrophorese werden die Gele mit Silbernitrat nach dem Protokoll von Bassam et al. (1990, Analytical Biochemistry 196, 80-83) gefärbt. Die Entwicklungsreaktion wird durch Überführen der Gele in eiskalte 0,04M EDTA-Lösung gestoppt.

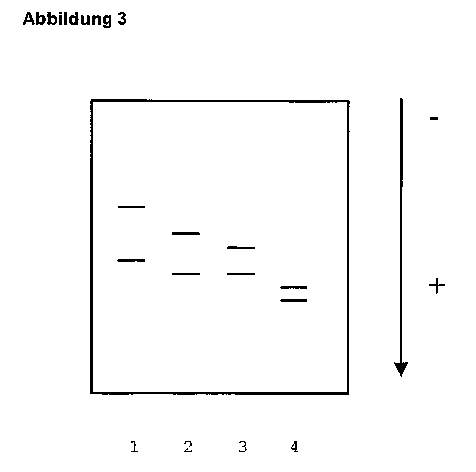

Das Wanderungsmuster der Allele 1 bis 4 zeigt schematisch Abbildung 3. Da mittels Silberfärbung beide DNS-Stränge (codierender und nicht-codierender DNS-Strang) angefärbt werden, sind jeweils zwei Fragmente pro Allel vorhanden.

### 2. Darstellung der Variabilität der Markers CSN1S1 bei verschiedenen Rinderrassen

Aus DNS von 83 Rindern der Rassen Deutsche Schwarzbunte (6 Rinder), Deutsches Rotvieh (4 Rinder), Gelbvieh (7 Rinder), Deutsch Holstein (18 Rinder), Fleckvieh (9 Rinder), Jersey (13 Rinder), Pinzgauer (20 Rinder) und Simbrah (6 Rinder) wird mit den Oligonukleotiden CSN1S1pro1f (5' GAA TGA ATG AAC TAG TTA CC 3') und CSN1S1pro1r (5' GAA GAA GCA GCA AGC TGG 3') die in Abbildung 1 dargestellte Nukleinsäuresequenz Position 1 bis 655 mittels PCR amplifiziert. Das weitere Vorgehen erfolgt wie unter Beispiel 1 beschrieben.

In den untersuchten Rassen tauchen die typischen, wie in Abbildung 3 gezeigten Auftrennungsmuster auf.

### 3. Darstellung der Variabilität der Markers CSN1S1 innerhalb der Rasse Deutsch Holstein

Aus Blutproben von 503 Kühen der Rasse Deutsch Holstein wird DNA nach der Methode von Mongomery & Sise (1990, NZ J Agric Res 33, 437-441) isoliert. Die Anreicherung der in Abbildung 2 dargestellten Sequenz erfolgt mit den Oligonukleotiden CSN1S1pro1f und CSN1S1pro1r wie oben beschrieben, die Darstellung der vorhandenen Variationen erfolgt mittels SSCP-Technik. Bei den untersuchten Kühen dieser Rasse sind alle ebenfalls vier Allele nachweisbar. Folgende Allelfrequenzen werden bestimmt:

| | |
|---|---|
| Allel 1 - 0,031 | Allel 2 - 0,739 |
| Allel 3 - 0,194 | Allel 4 - 0,036 |

Das Allel 2 stellt damit das häufigste Allel in Rasse Deutsch Holstein dar, gefolgt von Allel 3 und den zwei seltenen Allelen 1 und 4.

Die Genotypen treten in der Häufigkeit 22> 23 > 24 > 12 > 33 > 34 auf. Die Genotypen 11 und 14 sowie die Kombination dieser zwei seltenen Allele (Genotyp 14) werden bei den untersuchten Kühen nicht gefunden.

### 4. Genetische Kartierung des Markers CSN1S1

Mittels des erfindungsgemäßen Verfahren mit dem Marker *CSN1*S*1* werden acht Halbgeschwisterfamilien der Rassen Deutsch Holstein (7) und Fleckvieh (1) typisiert und mit den Ergebnissen von Thomsen et al. 2000 (J Anim Breed Genet 117, 289-306) verglichen, der diese Familien bereits für 10 weitere Marker auf BTA 6 (Mikrosatellitenmarker) typisiert und eine Kopplungskarte erstellt hat. Die Typisierungsdaten für CSN1S1 werden in diesen bestehenden Datensatz integriert. Die Kartierung unter Verwendung der Funktion BUILD des Programmpaketes CRI-MAP (Version 2.4; Green et al. 1990, Documentation of CRI-MAP, Washington School of Medicine, St. Louis, MO, USA) führt zu zwei möglichen Positionen des Markers CSN1S1: zwischen den Markern IL97 und FBN14 oder FBN14 und

CSN3. Die weiterhin durchgeführte FLIPS-Analyse führt zur endgültigen Kartierung von *CSN1S1* zwischen den Markern *FBN14* und *CSN3*. Die Gesamtlänge der mit den 11 Markern in den 8 Familien berechneten Kopplungskarte von BTA6 beträgt 161.1 cM. Die Position aller in die Kopplungskarte einbezogenen Marker und die vergleichenden Angaben aus den vorhandenen Genkarten MARC97 und IBRP97 zeigt die folgenden Tabelle 1. Dargestellt sind die Marker zur Erstellung der Kopplungskarte von BTA6, die Anzahl der informativen Meiosen und mit CRI-MAP berechnete Positionen (cM) auf der genetischen Karte (die

Karte ist mit "ADR" bezeichnet) im Vergleich zu den beiden veröffentlichten Genkarten MARC97 and IBRP97. Für die mit n.a. eingetragenen Marker ist keine Kartierung in den jeweiligen Genkarten angegeben.

**Tabelle 1**

| Marker | Informative | Position (cM) | | |
|---|---|---|---|---|
| | | ADR | MARC97 | IBRP97 |
| ILSTS93 | 193 | 0.0 | 0.0 | 16.0 |
| ILSTS90 | 156 | 28.5 | 11.8 | 0.0 |
| BM1329 | 141 | 56.8 | 35.5 | 45.0 |
| URB16 | 228 | 57.9 | n.a. | 40.0 |
| DIK82 | 356 | 78.5 | n.a. | 67.0 |
| ILSTS097 | 78 | 99.6 | 67.2 | 89.0 |
| FBN14 | 187 | 104.1 | n.a. | n.a. |
| CSN1S1 | 280 | 108.1 | (wie CSN3) | (wie CSN3) |
| CSN3 | 102 | 113.5 | 82.6 | 103.0 |
| BP7 | 208 | 123.6 | 91.2 | n.a. |
| BMC4203 | 186 | 161.1 | 112.9 | n.a. |

### 5. Varianzanalyse zur Schätzung von Effekten auf Milchleistungsmerkmale

Mittels des erfindungsgemäßen Verfahren werden insgesamt 729 Bullen aus 9 Halbgeschwisterfamilien der Rassen Deutsch Holstein und Simmental mit dem Marker *CSN1S1* typisiert. Die Verteilung der Genotypen in den 9 Halbgeschwisterfamilien zeigt Tabelle 2.

**Tabelle 2**

| Familie | *n* | *CSN1S1* Genotyp | | | | | | | |
|---|---|---|---|---|---|---|---|---|---|
| | | 12 | 13 | 14 | 22 | 23 | 24 | 33 | 34 |
| 1 | *19* | - | - | - | 9 | 10 | - | - | - |
| 2 | *108* | 48 | 5 | 5 | 37 | 9 | 4 | - | - |
| 3 | *106* | 4 | - | 3 | 40 | 10 | 37 | - | 12 |
| 4 | *27* | 12 | 3 | 2 | | 10 | - | - | - |
| 5 | *12* | - | 1 | - | 5 | 5 | - | 1 | - |
| 6 | *27* | - | - | - | 9 | 16 | 1 | 1 | |
| 7 | *55* | 1 | - | 1 | 22 | 4 | 23 | | 4 |
| 8 | *56* | 4 | 2 | - | 26 | 17 | 3 | 1 | 3 |
| 9 | *319* | 10 | - | - | 250 | 50 | 9 | | - |
| total | *729* | 79 | 11 | 11 | 398 | 131 | 77 | 3 | 19 |

Die Zuchtwerte der Bullen werden zentral durch die Vereinigten Informationssysteme Tierhaltung (VIT) in Verden geschätzt. Insgesamt gehen über 150.000 Töchter und deren Leistungsdaten in die Zuchtwertschätzung ein. Von allen Bullen werden deregressierte Zuchtwerte für die Milchmenge, Protein- und Fettmenge, Proteingehalt (in %) und Fettgehalt (in %) in der Varianzkomponentenschätzung verwendet. Die Deregression der Zuchtwerte erfolgt wie bei Thomsen et al. (2001, J Anim Breed Genet. 118, 357-370) beschrieben.

Die Varianzkomponentenschätzung wird mit dem Programmpaket SAS durchgeführt. Als einziger fixer Effekt wird zunächst der Marker CSN1 S1 im Modell berücksichtigt, da andere Einflussfaktoren (z.B. Betriebseffekte, Melkhäufigkeit) bereits im Rahmen der Zuchtwertschätzung korrigiert werden. Die Analyse ergibt signifikante Effekte des Markers CSN1 S1 auf alle untersuchten Merkmale (deregressierte Zuchtwerte für Proteingehalt (DRG_PP), Milchmenge (DRG_MY1), Fettmenge (DRG_FY1), Proteinmenge (DRG_PY1), Fettgehalt (DRG_FP)), Tabelle 3 zeigt den Effekt von CSN1S1 auf deregressierte Zuchtwerte für Milchleistungsmerkmale mit Angabe der Irrtumswahrscheinlichkeiten (p) für die Effekte auf die Einzelmerkmale.

**Tabelle 3**

| Merkmal | Irrtumswahrscheinlichkeit (p) |
|---|---|
| DRG-PP | < 0.0001 |
| DRG_MY1 | 0.0011 |
| DRG_FY1 | 0.0016 |
| DRG_PY1 | 0.0056 |
| DRG_FP | 0.0052 |

Die höchste Signifikanz wird für den Effekt auf DRG_PP berechnet. Da der untersuchte Marker CSN1S1 direkt im regulatorischen Bereich eines Milchproteingens liegt, könnte dies ein Hinweis auf einen direkten Effekt sein. Der Marker CSN1S1 erfüllt die Anforderungen an ein funktionelles Kandidatengen.

Die höchsten Zuchtwerte für Milchmenge (DRG_MY1) erzielen im Mittel Bullen mit dem Genotyp 12, wohingegen die höchsten Zuchtwerte für Proteingehalte (DRG_PP) in der Gruppe mit Genotyp 24 gefunden werden. Eine Zusammenstellung der *Least square* Mittelwerte (LS_means) für die Gruppen mit den Genotypen 12, 22, 23, und 24 zeigt Tabelle 4. Dargestellt sind die LS_means sowie Standardfehler für die deregressierten Zuchtwerte für Milchmenge (DRG_MY1) und Proteingehalt (DRG_PP) in Gruppen mit verschiedenen *CSN1S1* Genotypen.

**Tabelle 4**

| *CSN1S1* type | | LSMEAN ± se | |
|---|---|---|---|
| | n | DRG_MY1 | DRG_PP |
| 12 | 79 | 198.232 ± 15.700 | - 0.00022534 ± 0.00006470 |
| 22 | 398 | 155.341 ± 6.995 | - 0.00037495 ± 0.00002921 |
| 23 | 131 | 138.806 ± 12.192 | - 0.00038405 ± 0.00005271 |
| 24 | 76 | 112.364 ± 16.007 | 0.00008175 ± 0.00006650 |
| Alle | 684 | 152.353 | -0.000307 |

Zur genaueren Abklärung wird die Varianzanalyse innerhalb einzelner Familien und Gruppen von Familien mit denselben Genotypen erneut durchgeführt. Dabei zeigt sich, dass der Effekt auf die Milchmenge nicht in allen Familien bestätigt werden kann. In Familie 9, in welcher vom Vater ausschließlich das Allele 2 vererbt wird, ist der einzige verbleibende Effekt in der Nähe der 5% Signifikanzschwelle für DRG_PP zu finden (p = 0.0610). Weiterhin wird für alle Genotypgruppen und einzelne Familien ein Vergleich der LS-means für die Merkmale DRG_MY1, DRG_PP, DRG_FP durchgeführt und die Differenz der LS-means für die Genotypen 12, 23 und 24 zum häufigsten Genotyp 22 auf Signifikanz geprüft. Die Ergebnisse sind grafisch dargestellt in Abbildung 5.

### 6. Allelische Variabilität im Schaf

Genomische DNS von verschiedenen europäischen Schafrassen (Milch- und Fleischschaf) wird als Template zur Amplifikation der CSN1S1-5' Region wie vorher beschrieben eingesetzt und eine SSCP-Analyse durchgeführt, wobei 5 verschiedene Migrationsmuster auftreten, die dem Migrationsmuster von DNS aus

Rindern sehr ähnlich sind (Daten nicht gezeigt).

## Patentansprüche

1. Nukleotidsequenz bestehend aus Seq. ID No. 5, Seq ID No. 6, Seq. ID No. 7 oder Seq ID No. 8.

2. Verwendung einer Nukleotidsequenz gemäß Anspruch 1 zur Bestimmung des allelischen Zustands am 5'-Ende des αs1-Kaseingens.

3. Verwendung einer Nukleotidsequenz gemäß Anspruch 1 zur Genomanalyse, insbesondere zur Genkartierung und/oder Kopplungsanalyse.

4. Verwendung einer Nukleotidsequenz gemäß Anspruch 1 zur Erzeugung von Expressionsvektoren.

5. Verwendung einer Nukleotidsequenz gemäß Anspruch 1 zur Herstellung transgener Tiere.

6. Verwendung einer Nukleotidsequenz gemäß Anspruch 1 als Marker zur Zuchtauswahl von Rindern hinsichtlich Milchleistungsmerkmaler.

7. In vitro Verfahren zur Ermittlung des allelischen Zustandes des 5'-Endes des αs1-Kaseingens, **gekennzeichnet durch** die folgenden Schritte:
a) Bereitstellen des Ausgangsmaterials des zu untersuchenden Organismus,
b) Isolierung des genetischen Materials,
c) gezielte Isolierung oder Anreicherung einer Nukleotidsequenz gemäß Anspruch 1 oder eines Teilbereichs, innerhalb dessen variable Nukleotidpositionen zur Unterscheidung der Nukleotidsequenzen gemäß Anspruch 1 enthalten sind,
d) Nachweis des allelischen Zustandes im isolierten oder angereicherten Sequenzbereich

8. Verfahren gemäß Anspruch 7, **dadurch gekennzeichnet, dass** der Nachweis zur alters- und laktationsunabhängigen Untersuchung von Tieren auf Milchleistungsmerkmale erfolgt.

9. Verfahren gemäß Anspruch 7, **dadurch gekennzeichnet, dass** der Nachweis zur Selektion auf erhöhte Milchproteingehalte erfolgt.

10. Verfahren gemäß Anspruch 7, **dadurch gekennzeichnet, dass** zur Züchtung auf Milchmenge Rinder mit einem allelischen Zustand des Genotyps Seq. ID No. 5 und Seq. ID No. 6 des αs1-Kaseingens ausgewählt werden.

11. Verfahren gemäß Anspruch 7, **dadurch gekennzeichnet, dass** zur Züchtung auf Milchproteingehalt Rinder mit einem allelischen Zustand des Genotyps Seq. ID No. 6 und Seq. ID No. 8 des αs1-Kaseingens ausgewählt werden.

12. Verfahren gemäß Anspruch 7, **dadurch gekennzeichnet, dass** das Ausgangsmaterial Blut, Leukozyten, Gewebe, Sperma, einzelne Zellen, Zellmaterial aus Embryonen, eine Bakterienkultur oder auch isolierte Chromosomen ist.

13. Verfahren gemäß Anspruch 7, **dadurch gekennzeichnet, dass** das Ausgangsmaterial aus einem genetisch veränderten Organismus (GVO) stammt.

14. Verfahren gemäß Anspruch 7, **dadurch gekennzeichnet, dass** das genetische Material genomische DNA oder RNA von Tieren, Plasmid. DNA aus Bakterien oder von artifiziellen Chromosomen ist.

15. Verfahren gemäß Anspruch 7, **dadurch gekennzeichnet, dass** die Anreicherung der Nukleotidsequenz des αs1-Kaseingens in der PCR oder RT-PCR mit den Primern Seq. ID No. 1 und Seq. ID No. 2 erfolgt.

16. Verfahren gemäß Anspruch 7, **dadurch gekennzeichnet, dass** die Bestimmung des allelischen Zustands mittels SSCP, RFLP, OLA, TGGE, ASPCR, PCR-Elisa, Microarray-Verfahren oder durch Nukleinsäuresequenzierung erfolgt.

17. Verfahren gemäß Anspruch 7, **dadurch gekennzeichnet, dass** Organismen ausgewählt werden, die eine der Nukleotidsequenzen Seq. ID No. 5, Seq. ID No. 6. Seq. ID No. 7 oder Seq. ID No. 8 des 5'-Bereichs αs1-Kaseingens tragen.

18. Testkit zur Durchführung des Verfahrens gemäß einem der Ansprüche 7 -17 enthaltend:
- Primer Seq. ID No. 1 und Seq. ID No. 2 zur Anreicherung einer Nukleotidsequenz gemäß Anspruch 1 sowie
- Referenzproben für eine oder mehrere Sequenzen der Nukleotidsequenz des αs1-Kaseingens und dessen Allele

## Claims

1. A nucleotide sequence consisting of Seq. ID No. 5, Seq. ID No. 6, Seq. ID No. 7 or Seq. ID No. 8.

2. Use of a nucleotide sequence according to claim 1 to determine the allelic state at the 5' end of the αs1-casein gene.

3. Use of a nucleotide sequence according to claim 1 for genome analysis, in particular for gene mapping and/or linkage analysis.

4. Use of a nucleotide sequence according to claim 1 for the production of expression vectors.

5. Use of a nucleotide sequence according to claim 1 for the production of transgenic animals.

6. Use of a nucleotide sequence according to claim 1 as a marker for the selective breeding of cattle in connection with milk production traits.

7. In vitro method for ascertaining the allelic state of the 5' end of the αs1-casein gene, **characterized by** the following steps:
a) preparing the source material of the organism to be examined;
b) isolating the genetic material;
c) selective isolation or enrichment of a nucleotide sequence in accordance with claim 1 or a sub-domain thereof which contains variable nucleotides for distinguishing nucleotide sequences in accordance with claim 1;
d) typing the allelic state in the isolated or enriched sequence region.

8. The method according to claim 7, **characterized in that** typing is based on the animal's milk production traits, independently of age and lactation.

9. The method according to claim 7, **characterized in that** typing is based on raised milk protein levels.

10. The method according to claim 7, **characterized in that** in order to breed for milk yield, cattle are selected with an allelic state of the genotype Seq. ID No. 5 and Seq. ID No. 6 of the αs1-casein gene.

11. The method according to claim 7, **characterized in that** in order to breed for milk protein yield, cattle are selected with an allelic state of the genotype Seq. ID No. 6 and Seq. ID No. 8 of the αs1-casein gene.

12. The method according to claim 7, **characterized in that** the source material is blood, leukocytes, tissue, sperm, individual cells, cell material from embryos, a bacterial culture or isolated chromosomes.

13. The method according to claim 7, **characterized in that** the source material originates from a genetically modified organism (GMO).

14. The method of claim 7, **characterized in that** the genetic material is genomic DNA or RNA from animals, plasmids, DNA from bacteria or from artificial chromosomes.

15. The method of claim 7, **characterized in that** enrichment of the nucleotide sequence of the αs1-casein gene is carried out using PCR or RT-PCR with the primers Seq. ID No. 1 and Seq. ID No. 2.

16. The method of claim 7, **characterized in that** the allelic state is determined using SSCP, RFLP, OLA, TGGE, ASPCR, PCR-Elisa, microarray methods or by nucleic acid sequencing.

17. The method of claim 7, **characterized in that** organisms are selected which carry one of nucleotide sequences Seq. ID No. 5, Seq. ID No. 6, Seq. ID No. 7 or Seq. ID No. 8 of the 5' region of the αs1-casein gene.

18. A test kit for carrying out the method of one of claims 7 to 17, containing:
- primers Seq. ID No. 1 and Seq. ID No. 2 to enrich a nucleotide sequence in accordance with claim 1; and
- reference probes for one or more sequences of the nucleotide sequence of the αs1-casein gene and alleles thereof.

## Revendications

1. Séquence de nucléotides composée de seq. ID No. 5, seq. ID No. 6, seq. ID No. 7 ou de seq. ID No. 8.

2. Utilisation d'une séquence de nucléotides selon la revendication 1 pour déterminer l'état allélique à l'extrémité 5' du gène de la caséine αs1.

3. Utilisation d'une séquence de nucléotides selon la revendication 1 pour l'analyse génomique, notamment pour la cartographie génétique et/ou l'analyse de liaison génétique.

4. Utilisation d'une séquence de nucléotides selon la revendication 1 pour créer des vecteurs d'expression.

5. Utilisation d'une séquence de nucléotides selon la revendication 1 pour produire des animaux transgéniques.

6. Utilisation d'une séquence de nucléotides selon la revendication 1 comme marqueur dans l'élevage sélectif de bovins, ledit marqueur indiquant des propriétés de production laitière.

7. Procédé in vitro destiné à déterminer l'état allélique à l'extrémité 5' du gène des la caséine αs1, **caractérisé en ce qu'**il comporte les étapes suivantes
a) mise à disposition de la matière de départ issue de l'organisme soumis à l'analyse,
b) isolation du matériau génétique,
c) isolation ciblée ou enrichissement d'une séquence de nucléotides selon la revendication 1 ou d'un domaine faisant partie de cette dernière et contenant des nucléotides à emplacement variable qui permettent de différencier les séquences de nucléotides selon la revendication 1,
d) mise en évidence de l'état allélique dans le domaine de séquences isolé ou enrichi.

8. Procédé selon la revendication 7, **caractérisé en ce que** la mise en évidence est réalisée pour analyser les propriétés de production laitière des animaux indépendamment de leur âge et de leur état de lactation.

9. Procédé selon la revendication 7, **caractérisé en ce que** la mise en évidence est réalisée pour l'élevage sélectif visant à augmenter la teneur en protéines du lait.

10. Procédé selon la revendication 7, **caractérisé en ce qu'**on sélectionne les bovins dont le gène de la caséine αs1 présente un état allélique correspondant aux génotypes seq. ID No. 5 et seq. ID No. 6 pour un élevage visant à augmenter la production laitière.

11. Procédé selon la revendication 7, **caractérisé en ce qu'**on sélectionne les bovins dont le gène de la caséine αs1 présente un état allélique correspondant aux génotypes seq. ID No. 6 et Seq. ID No. 8 pour un élevage visant à augmenter la teneur en protéines du lait.

12. Procédé selon la revendication 7, **caractérisé en ce que** la matière de départ correspond à du sang, des leucocytes, du tissu, du sperme, des cellules isolées, des cellules issues d'embryons, à une culture bactérienne ou bien à des chromosomes isolés.

13. Procédé selon la revendication 7, **caractérisé en ce que** la matière de départ est issue d'un organisme génétiquement modifié (OGM).

14. Procédé selon la revendication 7, **caractérisé en ce que** le matériau génétique correspond à de l'ADN ou de l'ARN génomique d'origine animale, à de l'ADN plasmidique d'origine bactérienne où bien qu'il est issu de chromosomes artificiels.

15. Procédé selon la revendication 7, **caractérisé en ce que** l'enrichissement de la séquence de nucléotides du gène de la caséine αs1 est réalisée par PCR ou par RT-PCR avec les amorces seq. 1D No. 1 et seq. ID No. 2.

16. Procédé selon la revendication 7, **caractérisé en ce que** la détermination de l'état allélique est réalisée par SSCP, RFLP, OLA, TGGE, ASPCR, PCR-Elisa, par des procédés de microdosage ou par le séquençage d'acides nucléiques.

17. Procédé selon la revendication 7, **caractérisé en ce qu'**on choisit des organismes présentant l'une des séquences de nucléotides seq. ID No. 5, seq. ID No. 6, seq. ID No. 7 ou seq. ID No. 8 du domaine 5' du gène de la caséine αs-1.

18. Coffret d'analyse permettant la mise en oeuvre du procédé selon l'une des revendications 7 à 17 et contenant :
- les amorces seq. ID No. 1 et seq. ID No. 2 destinées à l'enrichissement d'une séquence de nucléotides selon la revendication 1 ainsi que
- des échantillons de référence pour une ou plusieurs séquences de la séquence de nucléotides du gène de la caséine αs1 ou de ses allèles.


### SEQUENCE LISTING

<110> TransMIT Gesellschaft für Technologietransfer mbH
<120> verfahren zur Bestimmung des allelischen zustandes am 5'-Ende des alphaS1-Kaseingens
<130> An127/Pri
<140> DE 102 38 433.9
   <141> 2002-08-16
<160> 8
<170> PatentIn version 3.3
<210> 1
   <211> 20
   <212> DNA
   <213> Bos spec.
<220>
   <221> Primer1
   <222> (1)..(20)
   <223> Länge: 20 Basenpaare
   Art: Nukleinsäure
   Strangform: einzel
   Topologie: linear
<400> 1
   gaatgaatga actagttacc 20
<210> 2
   <211> 18
   <212> DNA
   <213> Bos spec.
<220>
   <221> Primer 2
   <222> (1)..(18)
   <223> Länge: 18 Basenpaare
   Art: Nukleinsäure
   Strangform: einzel
   Topologie: linear
<400> 2
   gaagaagcag caagctgg 18
<210> 3
   <211> 19
   <212> DNA
   <213> Bos spec.
<220>
   <221> Primer 3
   <222> (1)..(19)
   <223> Länge: 19 Basenpaare
   Art: Nukleinsäure
   Strangform: einzel
   Topologie: linear
<400> 3
   ccttgaaata ttctaccag 19
<210> 4
   <211> 1061
   <212> DNA
   <213> Bos taurus
<220>
   <221> alpha-S1Kaseingen
   <222> (1)..(1061)
   <223> Beginn Exon 1 bei Position 620
<300>
   <301> Koczan Dirk, Hobom Gerd, seyfert Hans-Martin
   <302> Genomic organization of the bovine alpha S1-casein gene
   <303> Nucleic acids research
   <304> 19
   <305> 20
   <306> 5591
   <307> 1991-09-24
   <308> X59856
   <309> 1991-07-18
   <313> (1)..(1061)
<300>
   <308> EMBL X59856
   <309> 1991-07-18
   <313> (1)..(1061)
<400> 4
   gaatgaatga actagttacc acaactagta cacccaaaat gaacaaaaaa tagcttggtg 60
   gtataattaa aatgccacca aaatttatac aataattata ttttcttttt gcaggaaaaa 120
   gattagacca catataatgt aacttatttc acaaggtaaa taattataat aaataatatg 180
   gattaactga gttttaaaag gtgaaataaa taatgaattc ttctcatggt cttgtatgtt 240
   aataaaaatt gaaaaatttt gaagacccca ttttgtccca agaatttcat ttacaggtat 300
   tgaatttttc aaaggttaca aaggaaattt tattgatata ataaatgcat gttctcataa 360
   taaccataaa tctagggttt tgttggggtt tttttttgtt tgttaattta gaacaatgcc 420
   attccatttc ctgtataatg agtcacttct ttgttgtaaa ctctccttag aatttcttgg 480
   gagaggaact gaacagaaca ttgatttcct atgtgagaga attcttagaa tttaaataaa 540
   cctgttggtt aaactgaaac cacaaaatta gcattttact aatcagtagg tttaaatagc 600
   ttggaagcaa aagtctgcca tcaccttgat catcaaccca gcttgctgct tcttcccagt 660
   cttgggttca aggtattatg tatacatata acaaaatttc tatgattttc ctctgtctca 720
   tctttcattc ttcactaata cgcagttgta acttttctat gtgattgcaa gtattggtac 780
   tttcctatga tatactgtta gcttaaaaat atatttgcaa atgttgatac tatctatctc 840
   agagctatag gtgaaaaatt aaatactttt ataaagacca aattgatcat ttttaaacga 900
   aattcttata tactgaaaat gtagatacat aacttcagta tagatttatg gtaaaataat 960
   ttgaatcatt tttgtcaaat tctgtaaaaa gttgtcatac agaataattt ataatatttt 1020
   tgttttcata gaaataacat ttctggtaga atatttcaag g 1061
<210> 5
   <211> 652
   <212> DNA
   <213> Bos taurus
<220>
   <221> CSN1S1-Gen, 5 flankierende Region bis Position 616 und Exon 1 ab
   Position 617
   <222> (1)..(652)
   <223> Mutation/SNP Position 83 (A zu G), Position 98 (A zu G), Position 298 (A zu C), Position 442 (A zu G; Änderung/verlust einer YY1-und AP1-Bindungsstelle), Position 541 (G zu A);
   Deletion TT zwischen Position 389 und 394 verglichen mit Allel2
<400> 5
   gaatgaatga actagttacc acaactagta cacccaaaat gaacaaaaaa tagcttggtg 60
   gtataattaa aatgccacca aagtttatac aataattgta ttttcttttt gcaggaaaaa 120
   gattagacca catataatgt aacttatttc acaaggtaaa taattataat aaataatatg 180
   gattaactga gttttaaaag gtgaaataaa taatgaattc ttctcatggt cttgtatgtt 240
   aataaaaatt gaaaaatttt gaagacccca ttttgtccca agaatttcct ttacaggtat 300
   tgaatttttc aaaggttaca aaggaaattt tattgatata ataaatgcat gttctcataa 360
   taaccataaa tctagggttt tgttggggtt ttttgtttgt taatttagaa caatgccatt 420
   ccatttcctg tataatgagt cgcttctttg ttgtaaactc tccttagaat ttcttgggag 480
   aggaactgaa cagaacattg atttcctatg tgagagaatt cttagaattt aaataaacct 540
   attggttaaa ctgaaaccac aaaattagca ttttactaat cagtaggttt aaatagcttg 600
   gaagcaaaag tctgccatca ccttgatcat caacccagct tgctgcttct tc 652
<210> 6
   <211> 654
   <212> DNA
   <213> Bos taurus
<220>
   <221> CSN1S1-Gen, 5'flankierende Region und Exon 1
   <222> (1)..(654)
   <223> Bindungsstelle für Transkriptionsfaktor AP-1 bei Position 438 bis 445
   Bindungsstelle für Transkriptionsfaktor YY-1 bei Position 443 bis
   448
<400> 6
   gaatgaatga actagttacc acaactagta cacccaaaat gaacaaaaaa tagcttggtg      60
   gtataattaa aatgccacca aaatttatac aataattata ttttcttttt gcaggaaaaa     120
   gattagacca catataatgt aacttatttc acaaggtaaa taattataat aaataatatg     180
   gattaactga gttttaaaag gtgaaataaa taatgaattc ttctcatggt cttgtatgtt     240
   aataaaaatt gaaaaatttt gaagacccca ttttgtccca agaatttcat ttacaggtat     300
   tgaatttttc aaaggttaca aaggaaattt tattgatata ataaatgcat gttctcataa     360
   taaccataaa tctagggttt tgttggggtt ttttttgttt gttaatttag aacaatgcca      420
   ttccatttcc tgtataatga gtcacttctt tgttgtaaac tctccttaga atttcttggg     480
   agaggaactg aacagaacat tgatttccta tgtgagagaa ttcttagaat ttaaataaac     540
   ctgttggtta aactgaaacc acaaaattag cattttacta atcagtaggt ttaaatagct     600
   tggaagcaaa agtctgccat caccttgatc atcaacccag cttgctgctt cttc           654
<210> 7
   <211> 650
   <212> DNA
   <213> Bos taurus
<220>
   <221> CSN1S1-Gen, 5 flankierende Region
   <222> (1)..(650)
   <223> Bindungsstelle für Transkriptionsfaktor AP-1 bei Position 434 bis 441
   Bindungsstelle für Transkriptionsfaktor YY-1 bei Position 439 bis 444
   Deletion G und TTT zw. 390 und 396 verglichen mit Allel 2
<400> 7
   gaatgaatga actagttacc acaactagta cacccaaaat gaacaaaaaa tagcttggtg      60
   gtataattaa aatgccacca aaatttatac aataattata ttttcttttt gcaggaaaaa     120
   gattagacca catataatgt aacttatttc acaaggtaaa taattataat aaataatatg     180
   gattaactga gttttaaaag gtgaaataaa taatgaattc ttctcatggt cttgtatgtt     240
   aataaaaatt gaaaaatttt gaagacccca ttttgtccca agaatttcat ttacaggtat     300
   tgaatttttc aaaggttaca aaggaaattt tattgatata ataaatgcat gttctcataa     360
   taaccataaa tctagggttt tgttggggtt ttttttgtta atttagaaca atgccattcc      420
   atttcctgta taatgagtca cttctttgtt gtaaactctc cttagaattt cttgggagag     480
   gaactgaaca gaacattgat ttcctatgtg agagaattct tagaatttaa ataaacctgt     540
   tggttaaact gaaaccacaa aattagcatt ttactaatca gtaggtttaa atagcttgga     600
   agcaaaagtc tgccatcacc ttgatcatca acccagcttg ctgcttcttc                650
<210> 8
   <211> 650
   <212> DNA
   <213> Bos taurus
<220>
   <221> CSN1S1-Gen, 5 flankierende Region
   <222> (1)..(650)
   <223> Bindungsstellen für Transkriptionsfaktoren: AP-1 bei Position 434 bis 441, ABF1 bei Position 469 bis 483, YY-1 bei Position 439 bis 444;
   Mutation (SNP) in Position 480 (G zu C), damit entsteht eine
<400> 8
   gaatgaatga actagttacc acaactagta cacccaaaat gaacaaaaaa tagcttggtg     60
   gtataattaa aatgccacca aaatttatac aataattata ttttcttttt gcaggaaaaa    120
   gattagacca catataatgt aacttatttc acaaggtaaa taattataat aaataatatg    180
   gattaactga gttttaaaag gtgaaataaa taatgaattc ttctcatggt cttgtatgtt    240
   aataaaaatt gaaaaatttt gaagacccca ttttgtccca agaatttcat ttacaggtat    300
   tgaatttttc aaaggttaca aaggaaattt tattgatata ataaatgcat gttctcataa    360
   taaccataaa tctagggttt tgttggggtt ttttttgtta atttagaaca atgccattcc     420
   atttcctgta taatgagtca cttctttgtt gtaaactctc cttagaattt cttgggagac    480
   gaactgaaca gaacattgat ttcctatgtg agagaattct tagaatttaa ataaacctgt    540
   tggttaaact gaaaccacaa aattagcatt ttactaatca gtaggtttaa atagcttgga    600
   agcaaaagtc tgccatcacc ttgatcatca acccagcttg ctgcttcttc               650